# EUROPÄISCHE PATENTSCHRIFT

(11) **EP 1 501 546 B1**
(45) Veröffentlichungstag und Bekanntmachung des Hinweises auf die Patenterteilung: **10.10.2012**
(21) Anmeldenummer: 03725116.2
(22) Anmeldetag: 29.04.2003
(51) Int. Cl.: A61K 45/06, A61K 9/20, A61K 9/16, A61K 9/54, A61K 47/00

(54) **STABILE PHARMAZEUTISCHE FORMULIERUNG FÜR EINE KOMBINATION AUS EINEM STATIN MIT EINEM ACE-HEMMER**
STABLE PHARMACEUTICAL FORMULATION FOR A COMBINATION OF A STATIN AND AN ACE INHIBITOR
FORMULATION PHARMACEUTIQUE STABLE CONTENANT UNE STATINE COMBINEE AVEC UN INHIBITEUR DE L'ACE

(30) Priorität: 03.05.2002 DE 10219949; 16.05.2002 DE 10222326
(43) Veröffentlichungstag der Anmeldung: 02.02.2005
(73) Patentinhaber: Hexal AG, 83607 Holzkirchen (DE)
(72) Erfinder: HÄGER, Alexandra, 80335 München (DE); LUND HENRIKSEN, Kristian, DK-2860 Syborg (DK)
(74) Vertreter: Boeters, Hans Dietrich
(86) Internationale Anmeldenummer: PCT/EP2003/004472
(87) Internationale Veröffentlichungsnummer: WO 2003/092729

(56) Entgegenhaltungen:
- EP-A- 0 457 514
- WO-A-03/020243
- DE-A- 10 025 308
- MITAL SEEMA ET AL: "Simvastatin acts synergistically with ACE inhibitors or amlodipine to decrease oxygen consumption in rat hearts." JOURNAL OF CARDIOVASCULAR PHARMACOLOGY, Bd. 36, Nr. 2, August 2000 (2000-08), Seiten 248-254, XP002250967 ISSN: 0160-2446

## Beschreibung

Die Erfindung betrifft eine stabile pharmazeutische Formulierung, die eine Kombination aus einem Statin, wie Simvastatin, mit einem ACE-Hemmer, wie Ramipril, enthält.

Statine verhindern die Cholesterin-Biosynthese, indem sie hemmend auf das Enzym 3-Hydroxy-3-methylglutaryl-Coenzym A-Reduktase einwirken. Sie werden daher zur Behandlung der Hyperlipidämie eingesetzt. Zu den Statinen zählen Atorvastatin, Rosuvastatin, Fluvastatin, Pravastatin, Cerivastatin und insbesondere Simvastatin. Die Synthese von Simvastatin, (1S,3R,7S,8S,8aS)-1,2,3,7,8,8a-Hexahydro-3,7-dimethyl-8-{2-[(4R,6R)-tetrahydro-4-hydroxy-2-oxo-2H-pyran-6-yl]-ethyl}-1-naphthyl-2,2-dimethylbutyrat, wird beispielsweise in US 4,444,784 und EP 0 033 538 B1 dargestellt. Unter dem Namen ZOCOR^{R} ist Simvastatin als Filmtablette im Handel.

ACE-Hemmer wie Lisinopril, Enalapril, Quinapril, Imidapril, Fosinopril, Moexipril, Perindopril, Spirapril, Cilazapril, Captopril, Trandolapril und Ramipril wirken als Hemmstoffe auf das Angiotensin-Converting-Enzym. Sie werden zur Behandlung von essentieller Hypertonie und Herzinsuffizienz verwendet. In EP 0 079 022 B1 wird die Herstellung und pharmazeutische Verwendung von Ramipril, 2-{N-[(S)-1-(Ethoxycarbonyl)-3-phenylpropyl]-L-alanyl}-(1S,3S,5S)-2-azabicyclo[3.3.0]octan-3-carbonsäure, und seinen Salzen beschrieben.

Eine fixe Kombination eines Statins mit einem ACE-Hemmer in einer Darreichungsform hat den Vorteil, daß die Patienten-Compliance höher ist als bei getrennter Gabe. Bekanntlich ist die Einnahmezuverlässigkeit umso größer, je geringer die Anzahl der Präparate pro Einnahmezeitpunkt ist. Eine fixe Kombination verbessert somit die Sicherheit und Zuverlässigkeit einer Therapie.

In EP 0 457 514 B1 wird die Verwendung von ACE-Inhibitoren in Kombination mit einem cholesterinsenkenden Mittel, wie Pravastatin, zur Prophylaxe und Behandlung von Artherosklerose beschrieben.

EP 0 459 453 A2 offenbart die Verwendung von Pravastatin alleine oder in Kombination mit einem ACE-Inhibitor zur Vorbeugung oder Behandlung des Restenose-Risikos nach einer Angioplastie.

EP 0 461 548 A2 und EP 0 738 512 A1 beschreiben die Verwendung von HMG-CoA Reduktase-Inhibitoren alleine oder in Kombination mit einem ACE-Hemmer zur Verhinderung eines zweiten Herzinfarkts.

EP 0 482 498 A2 betrifft die Verwendung von cholesterinsenkenden Mitteln, wie Pravastatin, alleine oder in Kombination mit ACE-Inhibitoren, zur Vorbeugung und Behandlung von Diabetes.

In US 5,298,497 wird die Verwendung von cholesterinsenkenden Mitteln, wie Pravastatin, alleine oder in Kombination mit ACE-Inhibitoren, zur Verminderung des Bluthochdruckrisikos bei Patienten mit Insulinresistenz beschrieben.

WO 99/11260 betrifft die Kombination von Atorvastatin mit blutdrucksenkenden Mitteln, u.a. ACE-Inhibitoren.

WO 00/45818 offenbart die Behandlung von diabetischer Neuropathie mit Statinen, gegebenenfalls in Kombination mit ACE-Hemmern.

WO 01/15674 beschreibt die Verwendung beispielsweise von HMG-CoA Reduktase-Inhibitoren in Kombination mit ACE-Hemmern zur Vorbeugung oder Behandlung bestimmter Krankheiten.

WO 01/76573 betrifft den Einsatz beispielsweise von ACE-Inhibitoren mit cholesterinsenkenden Mitteln zur Vorbeugung von Herz-Kreislauf-Erkrankungen.

Es hat sich nun gezeigt, daß für eine fixe Statin-ACE-Hemmer-Kombination in einer oralen Darreichungsform die Stabilität der Wirkstoffe in der Tablette oder Kapsel den Anforderungen nicht genügt. Die ACE-Hemmer sind Verbindungen, die zu Zersetzungsreaktionen neigen (vgl. EP 0 280 999 B1), welche durch Säuren bzw. Basen begünstigt werden. Für eine Stabilisierung der empfindlichen Statine werden hingegen saure bzw. basische Substanzen eingesetzt. Daher erfolgt die Zersetzung des ACE-Hemmers in Gegenwart eines stabilisierten Statins in einem derart großen Ausmaß, daß schon nach kurzer Lagerzeit der Gehalt an den Zersetzungsprodukten so hoch ist, daß die Zulässigkeitsgrenze an Abbauprodukten weit überschritten wird.

Die Aufgabe der vorliegenden Erfindung ist die Bereitstellung einer pharmazeutischen Formulierung mit einem Statin in Kombination mit einem ACE-Hemmer, deren Stabilität bezüglich des Abbaus der Wirkstoffe den gesetzlichen Anforderungen entspricht. Der Gehalt an Wirkstoffen soll über einen längeren Zeitraum gleich bleiben und darf praktisch keinen Zersetzungsvorgängen unterworfen sein.

Die der Erfindung zugrundeliegende Aufgabe wird nun durch eine pharmazeutische Formulierung mit einem Gehalt an mindestens einem Statin und mindestens einem ACE-Hemmer gemäß Anspruch 1 gelöst, wobei das mindestens eine Statin und der mindestens eine ACE-Hemmer durch ein physiologisch verträgliches inertes Material getrennt sind.

Bei der erfindungsgemäßen pharmazeutischen Formulierung kann das mindestens eine Statin mit mindestens einem Statin-Stabilisator und der mindestens eine ACE-Hemmer mit mindestens einem fakultativen ACE-Hemmer-Stabilisator vorliegen.

Ferner kann die erfindungsgemäße pharmazeutische Formulierung mit einer Puffersubstanz und/oder einem Chelatbildner als Statin-Stabilisator vorliegen.

Ferner kann die erfindungsgemäße pharmazeutische Formulierung mit Vitamin E, Butylhydroxytoluol, Butylhydroxyanisol, Ascorbinsäure, Ascorbylpalmitat und/oder Natriumhydrogensulfit als Antioxidans vorliegen.

Ferner kann die erfindungsgemäße pharmazeutische Formulierung mit Dinatriumethylendiamintetraessigsäure, Kaliumcitrat, Natriumcitrat und/oder Citronensäure als Chelatbildner vorliegen.

Ferner kann die erfindungsgemäße pharmazeutische Formulierung mit einer Puffer-Substanz als ACE-Hemmer-Stabilisator vorliegen.

Ferner kann die erfindungsgemäße pharmazeutische Formulierung mit Natriumdihydrogenphosphat, Natriumcitrat, Natriumcarbonat, Natriumhydrogencarbonat und/oder Tris-(hydroxymethyl)-aminomethan als Puffersubstanz vorliegen.

Ferner kann die erfindungsgemäße pharmazeutische Formulierung mit einem natürlichen Polymeren, einem synthetischen Polymeren, einem nicht-einfachen Zucker und/oder einem anorganischen Material als inertes Material vorliegen.

Bei der erfindungsgemäßen pharmazeutischen Formulierung kann das trennende physiologisch verträgliche Material als Umhüllung der Kombination aus mindestens einem Statin und mindestens einem Statin-Stabilisator vorgesehen sein.

Ferner kann bei der erfindungsgemäßen pharmazeutischen Formulierung das mindestens eine Statin im Gemisch mit mindestens einem Statin-Stabilisator in Form von Partikeln vorliegen, insbesondere als Granulat oder Pellets, wobei die Partikel mit dem trennenden physiologisch verträglichem Material umhüllt sein können.

Ferner kann die erfindungsgemäße pharmazeutische Formulierung dadurch gewinnbar sein, dass man Partikel aus einem Gemisch von mindestens einem Statin und mindestens einem Statin-Stabilisator mit dem trennenden physiologisch verträglichen Material umhüllt.

Ferner kann die erfindungsgemäße pharmazeutische Formulierung dadurch gewinnbar sein, dass man das mindestens eine Statin und den mindestens einen Statin-Stabilisator in das trennende physiologisch verträgliche Material einbettet und dadurch umhüllt.

Ferner kann bei der erfindungsgemäßen pharmazeutischen Formulierung das mindestens eine Statin und der mindestens eine Statin-Stabilisator mit Sucrose, Metodextrin, Arabischem Gummi, Wachs, Polyvinylpyrrolidon, Gelatine, Cellulose, Cellulosederivat und/oder Shellak als inertem Material umhüllt sein.

Für die erfindungsgemäße pharmazeutische Formulierung können Hydroxpropylmethylcellulose, Hydroxypropylcellulose, Celluloseacetat und/oder Ethylcellulose als Cellulosederivat verwendet werden.

Bei der erfindungsgemäßen pharmazeutischen Formulierung können das mindestens eine Statin und der mindestens eine Statin-Stabilisator mit einem Polyacrylat, Polymethacrylat, Polyether und/oder Polyetheralkohol, insbesondere Polyethylenglycol, als Polymerem umhüllt sein.

Die erfindungsgemäße pharmazeutische Formulierung liegt in Form von Tabletten vor.

Die erfindungsgemäße pharmazeutische Formulierung ist in Form von Tabletten vorgesehen, bei denen das mindestens eine Statin und der mindestens eine ACE-Hemmer durch eine Schicht oder eine Membran aus einem physiologisch verträglichen inerten Material mit neutralem pH-Wert getrennt sind.

Für eine derartige erfindungsgemäße pharmazeutische Formulierung kann Calciumphosphat als anorganisches inertes Material vorgesehen sein.

Für eine derartige erfindungsgemäße pharmazeutische Formulierung kann auch mikrokristalline Cellulose und/oder pregelatinierte Stärke als inertes Material vorgesehen sein.

Eine erfindungsgemäße pharmazeutische Formulierung kann mit Atorvastatin, Rosuvastatin, Fluvastatin, Pravastatin, Cerivastatin und/oder Simvastatin als Statin vorgesehen sein.

Bei der erfindungsgemäßen pharmazeutischen Formulierung kann das mindestens eine Statin jeweils in Form der Carbonsäure, eines Carbonsäuresalzes, eines Lactons, eines Esters, eines Hydrats, eines Alkoholats und/oder eines Tautomeren vorliegen.

Eine erfindungsgemäße pharmazeutische Formulierung kann mit Captopril, Ceranapril, Zofenopril, Lisinopril, Enalapril, Quinapril, Imidapril, Fosinopril, Moexipril, Perindopril, Spirapril, Cilazapril, Trandolapril und/oder Ramipril als ACE-Hemmer vorliegen.

Bei der erfindungsgemäßen pharmazeutischen Formulierung kann der mindestens eine ACE-Hemmer jeweils in Form der Carbonsäure, eines Esters, eines Salzes, eines Säureadditions-Salzes, eines Hydrats und/oder eines Solvats als ACE-Hemmer vorliegen.

Überraschenderweise wurde nun gefunden, daß durch eine Umhüllung des stabilisierten Statins mit einer Schutzschicht eine Kombination mit einem ACE-Hemmer zu einer stabilen pharmazeutischen Formulierung möglich ist. Hierbei muß die Schutzschicht inert gegen die Stabilisatoren des Statins und fakultative Stabilisatoren des ACE-Hemmers sein.

Eine stabile pharmazeutische Formulierung kann auch durch eine Trennung von Statin und ACE-Hemmer mittels einer Membran erreicht werden.

Generell läßt sich sagen, daß die der Erfindung zugrundeliegende Aufgabe dadurch gelöst wird, daß die beiden Wirkstoffe - Statin und ACE-Hemmer - in einer pharmazeutischen Formulierung getrennt vorliegen.

Die erfindungsgemäße Wirkstoffkombination kann ein oder mehrere Statine, beispielsweise Atorvastatin, Rosuvastatin, Fluvastatin, Pravastatin, Cerivastatin und/oder Simvastatin enthalten. Die Statine können als Lacton, Ester (z.B. (C1-C4)-Alkylester), Carbonsäure bzw. als Salz der Carbonsäure, Hydrate, Alkoholate, Tautomere oder dergleichen eingesetzt werden.

Als bevorzugte Beispiele für Statin-Salze seien genannt: Alkalimetall-Salze wie Natrium- oder Kaliumsalzes sowie Ammoniumsalze, die abgeleitet sind von Ammoniak oder organischen Aminen, wie Ethylendiamin, Ethylamin, Diethylamin, Dipropylamin, Diisopropylamin, Tripropylamin, Trihexylamin, Tridodecylamin, Dicyclohexylamin, Ethanolamin, Diethanolamin, Triethanolamin, Triisopropanolamin, Arginin oder Lysin, sowie Erdalkalimetallsalze, wie Magnesium- oder Calciumsalze.

Bevorzugt wird das Simvastatin als Lacton verwendet.

Abbauprozesse der Statine können durch Temperatur, Feuchtigkeit, niedrigen pH-Wert, Licht und Sauerstoff begünstigt werden; vgl. etwa WO 00/35 425. Säureempfindliche Statine wie Atorvastatin, Rosuvastatin, Pravastatin, Fluvastatin und Cerivastatin werden daher im stark alkalischen Bereich bei pH 9 bis 10 formuliert. In diesen Fällen wirken Puffer als Stabilisatoren, vgl. etwa WO 00/35 425. Eine hinreichende Beständigkeit des Simvastatins kann mittels Stabilisatoren wie Antioxidantien und Chelatbildnern erzielt werden. Als Antioxidantien werden beispielsweise Vitamin E, Butylhydroxytoluol, Butylhydroxyanisol, Ascorbinsäure, Ascorbylpalmitat und/oder Natriumhydrogensulfit verwendet. Als Chelatbildner dienen z. B. Dinatriumethylendiamintetraessigsäure, Kaliumcitrat, Natriumcitrat und/oder Citronensäure. Bevorzugt wird zur Stabilisierung von Simvastatin Butylhydroxyanisol und Ascorbinsäure zusammen mit Citronensäure eingesetzt; vgl. Rote Liste.

Das (die) Statin(e) kann (können) zusammen mit dem (den) Stabilisator(en) und ggf. Hilfsstoffen nach den bekannten Verfahren granuliert oder pelletiert werden. Die Statin/Stabilisator-Partikel können demnach als Granulate oder Pellets vorliegen.

Die Statin/Stabilisator-Partikel haben beispielsweise einen Durchmesser von 44 µm bis 4 mm (325 bis 5 U.S. mesh), vorzugsweise von 300 bis 1000 µm.

Die Statin/Stabilisator-Partikel können mit einer Schutzumhüllung aus einem oder mehreren Polymeren versehen werden.

Die Schichtdicke der Schutzumhüllung beträgt beispielsweise wenige µm bis 90 µm, vorzugsweise etwa 20 µm.

Als Schutzumhüllung eignen sich alle physiologisch verträglichen Polymere, beispielsweise Metodextrin, Arabisches Gummi, Wachse, Gelatine, Polyvinylpyrrolidon, Acrylate, Methacrylate oder Polyethylenglykole sowie Cellulosederivate z.B. Hydroxypropylmethylcellulose, Hydroxypropylcellulose, Celluloseacetat, Ethylcellulose und/oder Shellac oder auch Sucrose. Bevorzugt werden Cellulosederivate verwendet. Es können auch Mischungen aus mehreren der genannten Stoffe eingesetzt werden.

Die umhüllten Statin/Stabilisator-Partikel können als Granulat oder Pellet, vorzugsweise als Granulat vorliegen.

Die umhüllten Statin/Stabilisator-Partikel können einen Durchmesser von 0.1 bis 2 mm, vorzugsweise von 0.15 bis 0.5 mm haben.

Eine Schutzumhüllung für die Statin/Stabilisator-Partikel kann mit Hilfe folgender Verfahren hergestellt werden: "fluidized-bed coating", "pan coating", Naß-, Zerstäubungs-, Aufbaugranulierung oder Kompaktierung.

"Fluidized-bed coating" erfolgt beispielsweise mittels der "top-spray"-, "Wurster-bottom-spray"- oder der "tangential-spray"-Methode (vgl. auch "Air suspension coating for multiparticulates", D. Jones, Drug Development and Industrial Pharmacy, 20(20), 1994, p. 3175-3206). Bei diesen Verfahren werden die Statin/Stabilisator-Partikel durch einen Luftstrom in Schwebe gehalten und mit dem/den Polymeren besprüht. Das (die) Polymer(en) und ggf. Hilfstoffe (s.u.) können in Lösung mit organischen Solventien und/oder Wasser, Suspension, Emulsion, Latex oder im geschmolzenen Zustand vorliegen.

Bei der "pan-coating"-Methode wird eine Lösung des (der) Polymeren in einen rotierenden Tiegel mit Statin/Stabilisator-Partikeln gesprüht.

Zur Naßgranulierung gehört das Mischen der Inhaltsstoffe wie Polymer(e), Statin/Stabilisator-Partikel, ggf. Hilfsstoffe und Solvens (bevorzugt Wasser) mit einem geeigneten Mischer oder Kneter (z. B. mit einem Planeten-, Pflugschar- oder Intensivmischer), Feuchtscreenen, Trocknen (z. B. in einem Wirbelschicht-, Umluft- oder Vakuumtrockner) und Trockenscreenen.

Für die Zerstäubungsgranulierung werden die Statin/Stabilisator-Partikel zunächst in einer Lösung des (der) Polymeren dispergiert. Anschließend wird diese Statin-PolymerSuspension in einem Trocknungs- oder Kühlturm zerstäubt. In einem Trocknungs- bzw. Kühlgas trocknen oder erstarren die erzeugten Tropfen zu einem Granulat. Eine Variante stellt das Zertropfen dar, bei dem aus einer Öffnung ein laminarer Flüssigkeitsfaden strömt, der durch regelmäßige Störungen eingeschnürt wird. Aufgrund der Oberflächenspannung bilden sich kugelförmige "droplets".

Bei der Aufbaugranulierung wird eine Lösung oder Suspension aus Statin(en), Stabilisator(en), Bindemitteln und/oder Hilfsstoffen auf einen inerten Kern, beispielsweise aus Zucker oder Cellulose, gesprüht. Die so erhaltene Wirkstoffschicht wird anschließend mit einer Polymer-Schutzschicht überzogen. Als Kern kann auch kristallines, stabilisiertes Statin dienen. Eine Aufbaugranulierung läßt sich mit Hilfe eines Tangential-Granulators durchführen.

Bei der Trockengranulierung (Kompaktierung) werden die Statin/Stabilisator-Partikel mit dem (den) Polymer(en) und ggf. Hilfsstoffen in Pulverform ohne Zusatz von Flüssigkeit unter hohem Druck granuliert.

Das (die) Statin(e) kann (können) auch mittels Extrusion gleichzeitig stabilisiert und umhüllt werden. Bei diesem Prozess wird das (die) Statin(e) mit dem (den) Polymeren, Stabilisator(en) und ggf. Hilfsstoffen vermischt, mit einer Granulierflüssigkeit versetzt, zu einer Masse mit bestimmter Plastizität homogenisiert und anschließend zu Pellets extrudiert.

Die erfindungsgemäße Wirkstoffkombination kann einen oder mehrere ACE-Hemmer, wie Captopril, Ceranapril, Zofenopril, Lisinopril, Enalapril, Quinapril, Imidapril, Fosinopril, Moexipril, Perindopril, Spirapril, Cilazapril, Trandolapril und/oder Ramipril enthalten. Bevorzugt wird Ramipril verwendet. Die ACE-Hemmer können als pharmazeutisch unbedenklichen Salze, Hydrate, Solvate sowie in Form von Derivaten eingesetzt werden. ACE-Hemmer in Form eines Esters, insbesondere eines C1-C4-Alkylesters, werden als Prodrugs verwendet, die dann im Körper in die aktiven Metaboliten (=Carbonsäuren) umgewandelt werden. Beispielsweise kann sowohl Ramipril, ein Ester-Prodrug, als auch sein aktiver Metabolit, die Dicarbonsäure Ramiprilat, als ACE-Hemmer genutzt werden.

Als Salze der ACE-Hemmer eignen sich beispielsweise Säureadditionssalze mit organischen oder anorganischen Säuren. Als organische Carbonsäuren kommen in Frage Salicylsäure, Maleinsäure, Weinsäure, Citronensäure, Adipinsäure, Sorbinsäure, Malonsäure, 1,4-Butandisäure, Äpfelsäure, Pivalinsäure, Bernsteinsäure, Nicotinsäure, Isonicotinsäure, Furan-2-carbonsäure, Essigsäure, Benzoesäure, Fettsäuren, wie z. B. Laurinsäure, Myristylsäure oder Ölsäure, als anorganische Säuren z. B. Salzsäure, Bromwasserstoffsäure, Iodwasserstoffsäure, Salpetersäure, Schwefelsäure und/oder Phosphorsäure.

Die ACE-Hemmer neigen zu Zersetzungsreaktionen wie Hydrolyse, Cyclisierung oder Oxidation (vgl. etwa EP 0 280 999 B1), welche durch Säuren oder Basen beschleunigt werden. Sie können mittels Puffersubstanzen wie Natriumdihydrogenphosphat, Natriumcitrat, Natriumcarbonat, Natriumhydrogencarbonat oder Tris-(hydroxymethyl)-aminomethan stabilisiert werden; vgl. etwa EP 0 317 878 B1.

Die Dosiseinheit der erfindungsgemäßen Kombination kann beispielsweise enthalten:
0.1 bis 100 mg, vorzugsweise 10 bis 80 mg, insbesondere 10 bis 40 mg Statin und 0.1 bis 500 mg, vorzugsweise 1 bis 50 mg, insbesondere 1 bis 15 mg ACE-Hemmer.

Als pharmazeutische Formulierung eignen sich Tabletten. Diese Darreichungsformen können Trägermaterialien, Füllstoffe, Bindemittel, Schmiermittel, Konservierungsmittel, Stabilisatoren, "bulking agent" (z. B. Mannitol), Aromen oder Farbpigmente enthalten.

Folgende Hilfsstoffe können bei der Granulat- bzw. Tablettenherstellung verwendet werden: Füllstoffe wie Cellulose und Cellulosederivate (z. B. mikrokristalline Cellulose), Zucker (z. B. Lactose, Fructose oder Dextrose), Zuckeralkohole (z. B. Mannitol oder Sorbitol), Stärke, anorganische Füllstoffe wie Calciumphosphat, Calciumsulfat, Natriumcalciumphosphat, Natriumchlorid oder Kaolin, Bindemittel wie Gelatine-, Stärke - oder Cellulosederivate (z. B. Hydroxypropylcellulose, Hydroxypropylmethylcellulose, Methylcellulose oder Natriumcarboxymethylcellulose), Polyvinylpyrrolidon, Zucker (z. B. Sucrose, Glucose oder Dextrose), natürliche Gummi (z. B. Natriumalginat, Arabisches Gummi oder Pektin), Sprengmittel wie Stärke und Stärkederivate (z. B. Natriumcarboxymethylstärke), quervernetztes Polyvinylpyrrolidon, unmodifizierte oder modifizierte Cellulose (z. B. Natriumcarboxymethylcellulose oder quervernetzte Carboxymethylcellulose), Algininate, Fließmittel wie mikrokristalline Cellulose, Schmiermittel wie Magnesiumstearat, Calciumstearat, Stearinsäure, Natriumstearylfumarat, Polyethylenglycol oder Palmitinsäure, Süßstoffe wie Saccharin oder Aspartam, Geschmacksstoffe wie Orange, Pfefferminz oder Kirsche.

Die Tabletten können einen Überzug aufweisen, beispielsweise aus Shellac, Zucker oder beidem.

ACE-Hemmer und nicht umhülltes Statin können zu einer Dreischichttablette verarbeitet werden. Die Statin-Schicht ist dann durch eine Membran von der Schicht des ACE-Hemmers getrennt.

Als Membranmaterialien eignen sich Hilfsstoffe mit neutralem pH-Wert, wie mikrokristalline Cellulose, pregelatinierte Stärke, Calciumphosphat und/oder deren Kombinationen.

Das umhüllte Statin kann mit dem ACE-Hemmer vermischt und nach den pharmazeutischen Standardverfahren zu Tabletten verpresst werden.

Eine Mischtablette aus Simvastatin und Ramipril zeigt eine raschere Freisetzung von Ramipril im Vergleich zu einer Tablette, die als Wirkstoff nur den ACE-Hemmer enthält.

Die Erfindung wird durch nachstehende Beispiele näher erläutert, ohne aber den Erfindungsumfang damit einzuschränken.

### Beispiel 1:

### Simvastatin-Schicht

| | |
|---|---|
| Simvastatin | 500,0 mg |
| Mikrokristalline Cellulose | 500,0 mg |
| Ethanol | 350,0 mg |
| Butylhydroxyanisol | 1,0 mg |
| Gereinigtes Wasser | 1250 mg |
| Ascorbinsäure | 125,0 mg |
| Citronensäure | 62,5 mg |
| Magnesiumstearat | 50,0 mg |
| Pregelatinierte Stärke | 80,0 mg |
| Lactose-Hydrat | 381,0 mg |

Mikrokristalline Cellulose und Simvastatin werden in einem "high-shear"-Mischer (Ultraturax) homogenisiert. Eine Lösung von Butylhydroxyanisol wird mit einer Lösung von Ascorbinsäure und Citronensäure in Wasser vereinigt. Diese Lösung wird zusammen mit der mikrokristallinen Cellulose und dem Simvastatin einer Naßgranulation in einem "high-shear"-Mischer unterworfen. Anschließend erfolgt eine Wirbelschichttrocknung bei 80 °C und <35 % RH. Die so erhaltenen Granulate werden durch ein 1.0 bis 1.5 mm Sieb gescreent und in einem Kegelmischer mit Magnesiumstearat granuliert.
Diese Granulate werden zusammen mit Lactose-Hydrat, pregelatinierte Stärke und Magnesiumstearat zu einer Simvastatin-Pressmasse verarbeitet.

### Ramipril-Schicht

| | |
|---|---|
| Ramipril | 10,0 mg |
| Natriumhydrogencarbonat | 8,0 mg |
| Hydroxypropylmethylcellulose | 30,0 mg |
| Mikrokristalline Cellulose | 96,8 mg |
| Pregelatinierte Stärke | 94,6 mg |
| Natriumstearylfumarat | 0,6 mg |

Ramipril und Natriumhydrogencarbonat werden mit Wasser naßgranuliert. Diese Granulate werden mit Methocel, mikrokristalliner Cellulose, Stärke und Natriumstearylfumarat zu einer Ramipril-Pressmasse verarbeitet.

### Dreischichttablette

Die Simvastatin-Schicht (1700 mg/Tablette) wird mit einer Schicht aus mikrokristalliner Cellulose (240 mg/Tablette) und der Ramipril-Schicht (240 mg/Tablette) auf einer Rundlauftablettenpresse zu einer Dreischichttablette verarbeitet.

### Beispiel 2:

### Simvastatin-Schicht

| | |
|---|---|
| Simvastatin | 40,0 mg |
| Mikrokristalline Cellulose | 20,0 mg |
| Butylhydroxyanisol | 0,08 mg |
| Ascorbinsäure | 10,0 mg |
| Citronensäure Monohydrat | 5,0 mg |
| Magnesiumstearat | 4,0 mg |
| Pregelatinierte Stärke | 40,0 mg |
| Lactose-Monohydrat | 281,0 mg |

Die Herstellung der Simvastatin-Pressmasse erfolgt wie in **Beispiel 1.**

### Ramipril-Schicht

| | |
|---|---|
| Ramipril | 10,0 mg |
| Natriumhydrogencarbonat | 10,0 mg |
| Hydroxypropylmethylcellulose | 25,0 mg |
| Mikrokristalline Cellulose | 97,0 mg |
| Pregelatinierte Stärke | 96,5 mg |
| Natriumstearylfumarat | 1,5 mg |

Die Verarbeitung zu einer Ramipril-Pressmasse erfolgt analog zu **Beispiel 1.**

### Dreischichttablette

Die Simvastatin-Schicht (400 mg/Tablette) wird mit einer Schicht aus mikrokristalliner Cellulose (240 mg/Tablette) und der Ramipril-Schicht (240 mg/Tablette) auf einer Rundlauftablettenpresse zu einer Dreischichttablette verarbeitet.

### Beispiel 3:

### Simvastatin-Schicht

| | |
|---|---|
| Simvastatin | 40,0 mg |
| Mikrokristalline Cellulose | 40,0 mg |
| Butylhydroxyanisol | 0,08 mg |
| Ascorbinsäure | 10,0 mg |
| Citronensäure | 5,0 mg |
| Magnesiumstearat | 4,0 mg |
| Pregelatinierte Stärke | 80,0 mg |
| Lactose-Monohydrat | 381,0 mg |

Die Herstellung der Simvastatin-Pressmasse erfolgt wie in **Beispiel 1.**

### Ramipril-Schicht

| | |
|---|---|
| Ramipril | 10,0 mg |
| Natriumhydrogencarbonat | 7,0 mg |
| Hydroxypropylmethylcellulose | 40,0 mg |
| Mikrokristalline Cellulose | 91,0 mg |
| Pregelatinierte Stärke | 91,0 mg |
| Magnesiumstearat | 1,0 mg |

Die Verarbeitung zu einer Ramipril-Pressmasse erfolgt analog zu **Beispiel 1.**

### Dreischichttablette

Die Simvastatin-Schicht (560 mg/Tablette) wird mit einer Schicht aus mikrokristalliner Cellulose (240 mg/Tablette) und der Ramipril-Schicht (240 mg/Tablette) auf einer Rundlauftablettenpresse zu einer Dreischichttablette verarbeitet.

### Beispiel 4:

### Simvastatin-Schicht

| | |
|---|---|
| Simvastatin | 40,0 mg |
| Mikrokristalline Cellulose | 20,0 mg |
| Butylhydroxyanisol | 0,08 mg |
| Ascorbinsäure | 10,0 mg |
| Citronensäure-Monohydrat | 5,0 mg |
| Magnesiumstearat | 4,0 mg |
| Pregelatinierte Stärke | 40,0 mg |
| Lactose Monohydrat | 281,0 mg |

Die Herstellung der Simvastatin-Pressmasse erfolgt wie in **Beispiel 1.**

### Ramipril-Schicht

| | |
|---|---|
| Ramipril | 10,0 mg |
| Natriumhydrogencarbonat | 10,0 mg |
| Hydroxypropylmethylcellulose | 35,0 mg |
| Mikrokristalline Cellulose | 91,5 mg |
| Pregelatinierte Stärke | 91,5 mg |
| Magnesiumstearat | 2,0 mg |

Die Verarbeitung zu einer Ramipril-Pressmasse erfolgt analog zu **Beispiel 1.**

### Dreischichttablette

Die Simvastatin-Schicht (400 mg/Tablette) wird mit einer Schicht aus mikrokristalliner Cellulose (240 mg/Tablette) und der Ramipril-Schicht (240 mg/Tablette) auf einer Rundlauftablettenpresse zu einer Dreischichttablette verarbeitet.

### Beispiel 5:

### Simvastatin-Schicht

| | |
|---|---|
| Simvastatin | 40,0 mg |
| Mikrokristalline Cellulose | 40,0 mg |
| Butylhydroxyanisol | 0,08 mg |
| Ascorbinsäure | 10,0 mg |
| Citronensäure | 5,0 mg |
| Magnesiumstearat | 4,0 mg |
| Pregelatinierte Stärke | 80,0 mg |
| Lactose-Monohydrat | 381,0 mg |

Die Herstellung der Simvastatin-Pressmasse erfolgt wie in **Beispiel 1.**

### Ramipril-Schicht

| | |
|---|---|
| Ramipril | 10,0 mg |
| Natriumhydrogencarbonat | 45,0 mg |
| Hydroxypropylmethylcellulose | 35,0 mg |
| Mikrokristalline Cellulose | 86,6 mg |
| Pregelatinierte Stärke | 81,0 mg |
| Magnesiumstearat | 1,2 mg |
| Stearinsäure | 1,2 mg |

Die Verarbeitung zu einer Ramipril-Pressmasse erfolgt analog zu **Beispiel 1.**

### Dreischichttablette

Die Simvastatin-Schicht (560 mg/Tablette) wird mit einer Schicht aus mikrokristalliner Cellulose (240 mg/Tablette) und der Ramipril-Schicht (260 mg/Tablette) auf einer Rundlauftablettenpresse zu einer Dreischichttablette verarbeitet.

## Patentansprüche

1. Pharmazeutische Formulierung in Form von Tabletten mit einem Gehalt an mindestens einem Statin und mindestens einem ACE-Hemmer, wobei das mindestens eine Statin und der mindestens eine ACE-Hemmer durch eine Schicht oder eine Membran aus einem physiologisch verträglichen inerten Material mit neutralem pH-Wert getrennt sind, und das mindestens eine Statin mit mindestens einem Statin-Stabilisator und gegebenenfalls der mindestens eine ACE-Hemmer mit mindestens einem ACE-Hemmer-Stabilisator vorliegen, wobei der Statin-Stabilisator Natriumdihydrogenphosphat, Natriumcitrat, Natriumcarbonat, Natriumhydrogencarbonat, Tris-(hydroxymethyl)-aminomethan, Vitamin E, Butylhydroxytoluol, Butylhydroxyanisol, Ascorbinsäure, Ascorbylpalmitat, Natriumhydrogen-sulfit und/oder ein Chelatbildner ist.

2. Pharmazeutische Formulierung nach Anspruch 1 mit Dinatriumethylendiamintetraessigsäure, Kaliumcitrat, Natriumcitrat und/oder Citronensäure als Chelatbildner.

3. Pharmazeutische Formulierung nach Anspurch 1 und/oder 2 mit einer Puffer-Substanz als ACE- Hemmer-Stabilisator.

4. Pharmazeutische Formulierung nach Anspruch 3 mit Natriumdihydrogenphosphat, Natrium- citrat, Natriumcarbonat, Natriumhydrogencarbonat und/oder Tris- (hydroxymethyl) - aminomethan als Puffersubstanz.

5. Pharmazeutische Formulierung nach mindestens einem der vorhergehenden Ansprüche mit einem natürlichen Polymeren, einem synthetischen Polymeren, einem nicht-einfachen Zucker und/oder einem anorganischen Material als inertes Material.

6. Pharmazeutische Formulierung nach mindestens einem der vorhergehenden Ansprüche, wobei das mindestens eine Statin im Gemisch mit mindestens einem Statin-Stabilisator in Form von Partikeln vorliegt, insbesondere als Granulate oder Pellets.

7. Pharmazeutische Formulierung nach Anspruch 6, wobei die Statin/Stabilisator-Partikel mit einer Schutzumhüllung aus einem oder mehreren Polymeren versehen sind.

8. Pharmazeutische Formulierung nach Anspruch 7, wobei die Schutzumhüllung ein physiologisch verträgliches Polymer ist, insbesondere Metodextrin, Arabischer Gummi, Wachs, Gelatine, Polyvinylpyrrolidon, Acrylat, Methacrylat, Polyethylenglycol, Cellulosederivat, Shellak und/oder Sucrose, oder Mischungen derselben.

9. Pharmazeutische Formulierung nach Anspruch 8 mit Hydroxpropylmethylcellulose, Hydroxypropylcellulose, Celluloseacetat und/oder Ethylcellulose als Cellulosederivat.

10. Pharmazeutische Formulierung nach mindestens einem der Ansprüche 1 bis 8, bei der das mindestens eine Statin und der mindestens eine Statin-Stabilisator mit einem Polyacrylat, Polymethacrylat, Polyether und/oder Polyetheralkohol, insbesondere Polyethylenglycol, als Polymerem umhüllt sind.

11. Pharmazeutische Formulierung nach einem der Ansprüche 1 bis 10 mit Calciumphosphat als Material für die Trennschicht oder Trennmembran.

12. Pharmazeutische Formulierung nach mindestens einem der Ansprüche 1 bis 10 mit mikrokristalliner Cellulose und/oder pregelatinierter Stärke als Material für die Trennschicht oder Trennmembran.

13. Pharmazeutische Formulierung nach mindestens einem der vorhergehenden Ansprüche mit Atorvastatin, Rosuvastatin, Fluvastatin, Pravastatin, Cerivastatin und/oder Simvastatin als Statin.

14. Pharmazeutische Formulierung nach mindestens einem der vorhergehenden Ansprüche und insbesondere nach Anspruch 13 mit mindestens einem Statin jeweils in Form der Carbonsäure, eines Carbonsäuresalzes, eines Lactons, eines Esters, eines Hydrats, eines Alkoholats und/oder eines Tautomeren.

15. Pharmazeutische Formulierung nach mindestens einem der vorhergehenden Ansprüche mit Captopril, Ceranapril, Zofenopril, Lisinopril, Enalapril, Quinapril, Imidapril, Fosinopril, Moexipril, Perindopril, Spirapril, Cilazapril, Trandolapril und/oder Ramipril als ACE-Hemmer.

16. Pharmazeutische Formulierung nach mindestens einem der vorhergehenden Ansprüche und insbesondere nach Anspruch 15 mit mindestens einem ACE-Hemmer jeweils in Form der Carbonsäure, eines Esters, eines Salzes, eines Säureadditions-Salzes, eines Hydrats und/oder eines Solvatsals ACE-Hemmer.

## Claims

1. A pharmaceutical formulation in tablet form having a content of at least one statin and at least one ACE inhibitor, wherein the at least one statin and the at least one ACE inhibitor are separated by a layer or a membrane of a physiologically acceptable inert material of neutral pH, and the at least one statin is present with at least one statin stabiliser and, optionally, the at least one ACE inhibitor is present with at least one ACE inhibitor stabiliser, wherein the statin stabiliser is sodium dihydrogen phosphate, sodium citrate, sodium carbonate, sodium hydrogen carbonate, tris(hydroxymethyl)aminomethane, vitamin E, butylated hydroxytoluene, butylated hydroxyanisole, ascorbic acid, ascorbyl palmitate, sodium hydrogen sulfite and/or a chelate-former.

2. The pharmaceutical formulation according to claim 1, having disodium ethylenediaminetetraacetic acid, potassium citrate, sodium citrate and/or citric acid as chelate-former.

3. The pharmaceutical formulation according to claim 1 and/or claim 2, having a buffer substance as ACE inhibitor stabiliser.

4. The pharmaceutical formulation according to claim 3, having sodium dihydrogen phosphate, sodium citrate, sodium carbonate, sodium hydrogen carbonate and/or tris(hydroxymethyl)aminomethane as buffer substance.

5. The pharmaceutical formulation according to at least one of the preceding claims, having a natural polymer, a synthetic polymer, a non-simple sugar and/or an inorganic material as inert material.

6. The pharmaceutical formulation according to at least one of the preceding claims, wherein the at least one statin is present in admixture with at least one statin stabiliser in the form of particles, particularly in the form of granules or pellets.

7. The pharmaceutical formulation according to claim 6, wherein the statin/ statin stabiliser - particles are provided with a protective coating of one or more polymers.

8. The pharmaceutical formulation according to claim 7, wherein the protective coating is a physiologically acceptable polymer, especially metodextrin, gum arabic, wax, gelatin, polyvinylpyrrolidone, acrylate, methacrylate, polyethylene glycol, a cellulose derivative, shellac and/or sucrose, or mixtures thereof.

9. The pharmaceutical formulation according to claim 8, having hydroxypropyl methylcellulose, hydroxypropyl cellulose, cellulose acetate and/or ethyl cellulose as cellulose derivative.

10. The pharmaceutical formulation according to at least one of the claims 1 to 8, wherein the at least one statin and the at least one statin stabiliser are encapsulated by a polyacrylate, polymethacrylate, polyether and/or polyether alcohol, especially polyethylene glycol, as polymer.

11. The pharmaceutical formulation according to any one of claims 1 to 10, having calcium phosphate as material for the separating layer or membrane.

12. The pharmaceutical formulation according to at least one of claims 1 to 10, having microcrystalline cellulose and/or pregelatinised starch as material for the separating layer or membrane.

13. The pharmaceutical formulation according to at least one of the preceding claims, having atorvastatin, rosuvastatin, fluvastatin, pravastatin, cerivastatin and/or simvastatin as statin.

14. The pharmaceutical formulation according to at least one of the preceding claims and especially according to claim 13, having at least one statin in each case in the form of the carboxylic acid, a carboxylic acid salt, a lactone, an ester, a hydrate, an alcoholate and/or a tautomer.

15. The pharmaceutical formulation according to at least one of the preceding claims, having captopril, ceranapril, zofenopril, lisinopril, enalapril, quinapril, imidapril, fosinopril, moexipril, perindopril, spirapril, cilazapril, trandolapril and/or ramipril as ACE inhibitor.

16. The pharmaceutical formulation according to at least one of the preceding claims and especially according to claim 15, having at least one ACE inhibitor in each case in the form of the carboxylic acid, an ester, a salt, an acid addition salt, a hydrate and/or a solvate as ACE inhibitor.

## Revendications

1. Formulation pharmaceutique sous forme de comprimés présentant une teneur en au moins une statine et au moins un inhibiteur d'ECA, dans laquelle la statine, au moins au nombre de une, et l'inhibiteur d'ECA, au moins au nombre de un, sont séparés par une couche ou une membrane faite d'un matériau inerte physiologiquement compatible et présentant une valeur de pH neutre, et dans laquelle ladite statine, au moins au nombre de une, s'accompagne d'au moins un agent de stabilisation de la statine, et le cas échéant, ledit inhibiteur d'ECA, au moins au nombre de un, d'au moins un agent de stabilisation de l'inhibiteur d'ECA, dans laquelle l'agent de stabilisation de la statine est le dihydrogénophosphate de sodium, le citrate de sodium, le carbonate de sodium, l'hydrogénocarbonate de sodium, le tris-(hydroxyméthyl)-aminométhane, la vitamine E, le butylhydroxytoluène, le butylhydroxyanisol, l'acide ascorbique, le palmitate d'ascorbyle, l'hydrogénosulfite de sodium et/ou un agent chélatant.

2. Formulation pharmaceutique selon la revendication 1, comprenant de l'acide disodium-éthylènediaminetétraacétique, du citrate de potassium, du citrate de sodium et/ou de l'acide citrique en tant qu'agent chélatant.

3. Formulation pharmaceutique selon la revendication 1 et/ou 2, comprenant une substance tampon en tant qu'agent de stabilisation de l'inhibiteur d'ECA.

4. Formulation pharmaceutique selon la revendication 3, comprenant du dihydrogénophosphate de sodium, du citrate de sodium, du carbonate de sodium, de l'hydrogénocarbonate de sodium et/ou du tris-(hydroxyméthyl)-aminométhane en tant que substance tampon.

5. Formulation pharmaceutique selon au moins l'une des revendications précédentes, comprenant un polymère naturel, un polymère synthétique, un sucre non simple et/ou un matériau inorganique en tant que matériau inerte.

6. Formulation pharmaceutique selon au moins l'une des revendications précédentes, dans laquelle la statine, au moins au nombre de une, se présente en mélange avec au moins un agent de stabilisation de la statine sous forme de particules, en particulier sous forme de granulés ou de pastilles.

7. Formulation pharmaceutique selon la revendication 6, dans laquelle les particules statine/agent de stabilisation comportent une enveloppe protectrice composée d'un ou de plusieurs polymères.

8. Formulation pharmaceutique selon la revendication 7, dans laquelle l'enveloppe protectrice est un polymère physiologiquement compatible, en particulier une métodextrine, de la gomme arabique, de la cire, de la gélatine, de la polyvinylpyrrolidone, un acrylate, un méthacrylate, un polyéthylèneglycol, un dérivé de cellulose, de la gomme-laque et/ou du saccharose, ou des mélanges de ceux-ci.

9. Formulation pharmaceutique selon la revendication 8, comprenant de l'hydroxypropylméthylcellulose, de l'hydroxypropylcellulose, de l'acétate de cellulose et/ou de l'éthylcellulose en tant que dérivé de cellulose.

10. Formulation pharmaceutique selon au moins l'une des revendications 1 à 8, dans laquelle la statine, au moins au nombre de une, et l'agent de stabilisation de la statine, au moins au nombre de un, sont enveloppés d'un polyacrylate, d'un polyméthacrylate, d'un polyéther et/ou d'un alcool de polyéther, en particulier de polyéthylèneglycol, en tant que polymère.

11. Formulation pharmaceutique selon l'une des revendications 1 à 10, comprenant du phosphate de calcium en tant que matériau de la couche de séparation ou de la membrane de séparation.

12. Formulation pharmaceutique selon au moins l'une des revendications 1 à 10, comprenant de la cellulose microcristalline et/ou de l'amidon prégélatinisé en tant que matériau de la couche de séparation ou de la membrane de séparation.

13. Formulation pharmaceutique selon au moins l'une des revendications précédentes, comprenant de l'atorvastatine, de la rosuvastatine, de la fluvastatine, de la pravastatine, de la cérivastatine et/ou de la simvastatine en tant que statine.

14. Formulation pharmaceutique selon au moins l'une des revendications précédentes et en particulier selon la revendication 13, comprenant au moins une statine respectivement sous la forme d'acide carboxylique, d'un sel d'acide carboxylique, d'une lactone, d'un ester, d'un hydrate, d'un alcoolate et/ou d'un tautomère.

15. Formulation pharmaceutique selon au moins l'une des revendications précédentes, comprenant du captopril, du céranapril, du zéfonapril, du lisinopril, de l'énalapril, du quinapril, de l'imidapril, du fosinopril, du moexipril, du périndopril, du spirapril, du cilazapril, du trandolapril et/ou du ramipril en tant qu'inhibiteur d'ECA.

16. Formulation pharmaceutique selon au moins l'une des revendications précédentes, et en particulier selon la revendication 15, comprenant au moins un inhibiteur d'ECA respectivement sous la forme d'acide carboxylique, d'un ester, d'un sel, d'un sel d'addition d'acide, d'un hydrate et/ou d'un solvate en tant qu'inhibiteur d'ECA.
